(19) Europäisches Patentamt

European Patent Office

Office européen des brevets

(11) **EP 1 317 423 B1**

(12) **FASCICULE DE BREVET EUROPEEN**

(45) Date de publication et mention
de la délivrance du brevet:
**20.04.2005 Bulletin 2005/16**

(21) Numéro de dépôt: **01967465.4**

(22) Date de dépôt: **10.09.2001**

(51) Int Cl.7: **C07C 303/16**, C07C 309/04

(86) Numéro de dépôt international:
**PCT/FR2001/002799**

(87) Numéro de publication internationale:
**WO 2002/022564 (21.03.2002 Gazette 2002/12)**

(54) **PROCEDE DE FABRICATION DE CHLORURES D'ALCANESULFONYLE**

VERFAHREN ZUR HERSTELLUNG VON ALKANSULFONYLCHLORIDEN

METHOD FOR MAKING ALKANESULPHONYL CHLORIDES

(84) Etats contractants désignés:
**AT BE CH CY DE DK ES FI FR GB GR IE IT LI LU
MC NL PT SE TR**

(30) Priorité: **13.09.2000 FR 0011648**

(43) Date de publication de la demande:
**11.06.2003 Bulletin 2003/24**

(73) Titulaire: **ARKEMA
92800 Puteaux (FR)**

(72) Inventeurs:
• **OLLIVIER, Jean
F-64260 Arudy (FR)**
• **HAURAT, Gisèle
F-64370 Arthez de Bearn (FR)**

(74) Mandataire: **Granet, Pierre
ARKEMA
Département Propriété Industrielle
4-8, cours Michelet,
La Défense 10
92091 Paris La Défense Cedex (FR)**

(56) Documents cités:
**FR-A- 1 598 279         FR-A- 2 083 422
US-A- 3 248 423          US-A- 3 600 136**

• **B.D. CHRISTIE, ET AL.: "Synthesis of optically
pure pipecolates from L-asparagine. Application
to the total synthesis of (+)-apovincamine
through amino acid decarbonylation and
iminium ion cyclisation" JOURNAL OF
ORGANIC CHEMISTRY, vol. 50, no. 8, 19 avril
1985 (1985-04-19), pages 1239-1246,
XP002168706 American Chemical Society,
Washington, DC, US ISSN: 0022-3263**

## Description

La présente invention concerne le domaine des chlorures d'alcanesulfonyle et a plus particulièrement pour objet leur synthèse par chlorolyse oxydante du mercaptan correspondant en présence d'eau selon la réaction globale :

$$RCH_2\text{-}SH + 2\ H_2O + 3\ Cl_2 \rightarrow RCH_2\text{-}SO_2Cl + 5\ HCl$$

La consultation de l'art antérieur montre que cette synthèse des chlorures d'alcanesulfonyle à partir de mercaptans s'effectue, en régime discontinu ou en régime continu, en mettant en contact le chlore et le mercaptan au sein d'une solution aqueuse concentrée d'acide chlorhydrique (brevets FR 1 598 279, DE 1 811 768, US 3 626 004, JP 52-020970, US 3 600 136 et US 3 993 692) ou d'eau pure (brevets DE 2 845 918, JP 52-008283, US 3 248 423 et US 4 280 966), l'objectif étant d'obtenir un chlorure d'alcanesulfonyle avec un bon rendement et la meilleure pureté possible, c'est-à-dire en évitant la formation de composés secondaires ou intermédiaires gênants. Parmi ces composés, l'un des plus gênants est le chlorure de 1-chloro-alcanesulfonyle RCHCl-SO$_2$Cl; on trouve également l'acide alcanesulfinique RCH$_2$-SO$_2$H, l'alcanethiosulfonate d'alkyle RCH$_2$-SO$_2$-SCH$_2$R et le chlorure d'alcanesulfinyle RCH$_2$-SOCl, éventuellement associés à leur dérivé α-chloré. Ces composés, pour la plupart peu stables, sont à l'origine de la coloration du chlorure d'alcanesulfonyle au cours du temps.

Afin d'éviter la formation de tels composés, il est avantageux de travailler avec un excès d'eau. Ceci permet de limiter considérablement la formation de chlorure de 1-chloro-alcanesulfonyle et on ne trouve plus d'acide alcanesulfinique, ni d'alcanethiosulfonate d'alkyle ou de chlorure d'alcanesulfinyle dans le chlorure d'alcanesulfonyle préparé de cette manière.

L'acide chlorhydrique produit par la réaction reste en majeure partie piégé dans l'eau qu'il est nécessaire d'introduire en quantités importantes pour assurer une bonne sélectivité en chlorure d'alcanesulfonyle. C'est l'une des raisons pour laquelle les brevets précités FR 1 598 279, DE 1 811 768, US 3 626 004, JP 52-020970, US 3 600 136 et US 3 993 692 préconisent d'amener l'eau sous forme de solution aqueuse d'acide chlorhydrique concentré, de façon à récupérer un gaz chlorhydrique pur, les impuretés étant piégées en majeure partie dans la phase aqueuse acide.

Cependant, comme expliqué plus haut, on favorise ainsi la formation du dérivé α-chloré RCHCl-SO$_2$Cl, une impureté gênante pour les applications du chlorure RCH$_2$-SO$_2$Cl et très difficile à séparer par les techniques courantes de purification.

On connaît également par le brevet FR 2 083 422 un procédé de préparation d'un chlorure de sulfonyle de perfluoroalkyl-alkylène comprenant l'ajout à un composé précurseur thiol (ou mercaptan), en milieu aqueux, de préférence en dispersion aqueuse, de chlore jusqu'à saturation. La dispersion aqueuse est avantageusement faiblement acidifiée avant réaction.

De l'analyse de la technique antérieure, il ressort que les procédés proposés ne prennent pas en compte la qualité de l'acide chlorhydrique coproduit et qu'on ne s'est pas intéressé à sa valorisation. Récupéré après décantation du milieu réactionnel, il a accumulé différents sous-produits comme l'acide sulfurique, les acides sulfinique et sulfonique, un peu de chlorure d'alcanesulfonyle, etc ..., et il est donc impropre en l'état à la commercialisation.

En résumé :

1) lorsque l'eau est apportée sous forme d'une solution aqueuse concentrée d'acide chlorhydrique, on récupère un HCl gazeux de bonne qualité, mais le chlorure d'alcanesulfonyle obtenu contient des quantités relativement importantes du dérivé a-chloré RCHCl-SO$_2$Cl;

2) lorsque l'eau est apportée pure, le chlorure d'alcanesulfonyle est de très bonne qualité (très peu de chlorure de 1-chloro-alcanesulfonyle), mais la phase aqueuse récupérée est polluée par de l'acide sulfurique et des composés organiques, et la concentration de l'acide chlohydrique obtenu ne correspond pas forcément à une spécification commerciale.

La présente invention a donc pour but de cumuler les avantages respectifs des procédés à l'eau pure et des procédés utilisant une solution aqueuse concentrée d'acide chlorhydrique, de façon à obtenir à la fois un chlorure d'alcanesulfonyle de très bonne qualité et un acide chlorhydrique de qualité commerciale.

Il a maintenant été trouvé que ce but peut être atteint en dispersant préalablement de l'eau dans le mercaptan et en injectant la dispersion ainsi obtenue dans le mélange réactionnel.

L'invention a donc pour objet un procédé de fabrication d'un chlorure d'alcanesulfonyle RCH$_2$-SO$_2$Cl par chlorolyse oxydante du mercaptan RCH$_2$-SH correspondant, caractérisé en ce que le mercaptan et de l'eau sont introduits dans le mélange réactionnel sous forme d'une dispersion d'eau dans le mercaptan, le dit mélange réactionnel étant initialement composé d'une solution aqueuse concentrée d'acide chlorydrique.

Cette dispersion peut être obtenue, par exemple, par agitation mécanique, par un mélangeur statique ou par tout autre moyen connu de l'homme de l'art pour obtenir une dispersion fine d'eau dans la phase organique de mercaptan. La dispersion peut éventuellement être stabilisée par l'ajout d'une petite quantité (500 à 1000 ppm par rapport au poids d'eau d'appoint) d'un tensio-actif tel que, par exemple, l'acide octanesulfonique, l'acide décanesulfonique, l'acide dodécanesulfonique ou le sel de sodium d'un tel acide.

Bien que le rapport molaire eau:mercaptan puisse aller de 0,5:1 à 5:1, la quantité d'eau dispersée dans le mercaptan est avantageusement celle qui correspond à la stoechiométrie de la réaction mentionnée plus haut, soit deux moles d'eau par mole de mercaptan. Avec un rapport molaire eau:mercaptan égal à 2:1, la teneur massique en eau de la dispersion variera par exemple de 28,5 % pour le butylmercaptan à 17,1 % pour le décylmercaptan (23,3 % pour l'hexylmercaptan, 19,7 % pour l'octylmercaptan, etc... ).

Conformément au procédé selon l'invention, la dispersion d'eau dans le mercaptan est ensuite injectée dans le mélange réactionnel rendu aussi homogène que possible et composé initialement d'une solution concentrée d'acide chlorhydrique dans l'eau (33 à 40 %). La concentration du mélange réactionnel en chlorure d'alcanesulfonyle peut aller de 0 à 50 %; elle varie selon le degré d'avancement de la réaction et le mode de fonctionnement.

Le procédé selon l'invention peut être mis en oeuvre en mode discontinu (batch) ou en mode continu. En mode discontinu, on place préalablement dans le réacteur un pied d'acide chlorhydrique (36 à 40 %), puis sous forte agitation on introduit progressivement la dispersion eau/mercaptan et le chlore avec un ratio molaire chlore : mercaptan préférentiellement égal à 3:1. L'acide chlorhydrique gazeux produit pendant la réaction est abattu à l'eau dans une colonne de type bien connu de l'homme de métier et le chlorure d'alcanesulfonyle produit est récupéré après décantation et séparation de la phase aqueuse acide qui peut éventuellement être recyclée pour une autre opération.

La mise en oeuvre du procédé selon l'invention en mode continu nécessite deux réacteurs en série, le premier (réacteur principal) effectuant l'essentiel de la réaction et le second (réacteur finisseur) servant à éliminer les intermédiaires non oxydés. On crée un tourne-en-rond d'acide chlorhydrique (33 à 40 % dans l'eau), qui va conditionner le temps de séjour dans le réacteur principal et le finisseur. On introduit les réactifs dans le réacteur principal de la même manière qu'en mode discontinu. En aval du réacteur principal, un séparateur gaz-liquide permet de récupérer le gaz chlorhydrique, abattu ensuite à l'eau dans une colonne prévue à cet effet. Le réacteur finisseur, lui aussi agité, reçoit un appoint de chlore et son contenu passe ensuite dans un décanteur pour séparer le sulfochlorure de la solution aqueuse d'acide chlorhydrique concentrée que l'on recycle à l'aide d'une pompe.

Le procédé selon l'invention peut s'appliquer à la synthèse des chlorures d'alcanesulfonyle $RCH_2$-$SO_2Cl$ contenant de 2 à 12 atomes de carbone, de préférence 4 à 10. Le radical $RCH_2$ est de préférence un radical alkyle linéaire, mais il peut également être ramifié comme, par exemple, dans le cas de l'isobutylmercaptan.

Les exemples suivants dans lesquels les pourcentages indiqués sont des pourcentages massiques, illustrent l'invention sans la limiter.

Les essais ont été réalisés dans un dispositif comprenant un réacteur d'une capacité de 10 litres, équipé d'une double enveloppe branchée sur un groupe froid, d'un condenseur, d'une prise de température et d'un agitateur mécanique comportant deux mobiles et pouvant tourner de 250 à 1000 tours par minute. L'alimentation en gaz (chlore ou azote) est effectuée via une canalisation par l'intermédiaire d'un fritté de verre. Le débit du gaz est contrôlé par un débitmètre massique à commande électronique. Deux pompes doseuses injectent respectivement le mercaptan et l'eau d'appoint dans un mélangeur statique, placé directement dans le milieu réactionnel pour que l'émulsion mercaptan/eau n'ait pas le temps de démixer.

Le gaz sortant du condenseur est amené dans une colonne d'abattage à l'eau de l'acide chlorhydrique produit par la réaction. Un rotamètre mesure le débit gazeux total en sortie du réacteur et la teneur en chlore de l'effluent gazeux sortant du réacteur est mesurée à l'aide d'un spectrophotomètre UV branché en ligne.

**Exemple 1 : Préparation du chlorure de n-octanesulfonyle en mode discontinu**

Dans le réacteur, on a préalablement placé 5200 grammes (4,41 litres) d'une solution aqueuse d'acide chlorhydrique 36 % et, après avoir amené cette solution à 6°C sous forte agitation, on a mis en route l'introduction de la dispersion eau / n-octylmercaptan (ratio molaire 2/1) et, en même temps, celle de chlore de façon à ce que le ratio molaire chlore/mercaptan soit égal à 3/1.

Pour transformer 3000 grammes de n-octylmercaptan en chlorure de n-octanesulfonyle, on a introduit 4362 grammes de chlore (61,5 moles) et 738 grammes d'eau (41 moles). La température montée progressivement à 20°C a été maintenue à ce niveau grâce à la double enveloppe réfrigérée.

Après avoir introduit en 5 heures toute la quantité de n-octylmercaptan désirée (3000g ), on a arrêté son alimentation et celle de l'eau d'appoint, mais on a continué durant une heure d'injecter du chlore à seulement 5 % de son débit initial, en maintenant l'agitation, afin de parachever l'oxydation des intermédiaires de synthèse en chlorure d'octanesulfonyle.

Dans le réacteur, on a récupéré 9974 g d'un mélange biphasique composé de 4362 g de chlorure de n-octanesulfonyle brut, 66 g de HCl dissous dans ce chlorure et 5546 g d'une solution d'acide chlorhydrique 40 %. Une heure de décantation a permis de séparer les deux phases organique et aqueuse.

Après stripping de l'HCl et des traces de chlore qu'il contient, l'analyse de la phase organique a fourni les résultats suivants :

- chlorure d'octanesulfonyle : 97 % (production : 4231 g)
- chlorure de 1-chlorooctanesulfonyle : 0,25 %
- chlorure de 1-chlorooctanesulfinyle : 0,1 %
- octanethiosulfonate d'octyle : 0,1 %
- eau : 0,1 %
- acide octanesulfonique : 0,2 %
- acide octanesulfinique : 0,1 %
- chlorure d'iso-octanesulfonyle : 0,3 % (provient de l'isomercaptan contenu dans le réactif de départ)
- autres lourds : 1,85 %

Pour une production totale de 3739 g d'acide chlorhydrique, le bilan s'est établi comme suit :

- 65,44 g dissous dans le chlorure d'octanesulfonyle brut
- 346,7 g piégés dans la phase aqueuse acide qu'ils amènent à un titre de 40 %
- 3327 g (soit 89 % de l'acide produit) abattus à l'eau pour faire de l'acide à 33 % (9981 g)

La phase aqueuse acide (5546 g) avait la composition suivante :

- titre HCl : 40 %
- chlorure d'octanesulfonyle : 0,20 %
- acide sulfurique : 0,26 %
- acide octanesulfonique : 0,074 %
- acide octanesulfinique : 0,023 %

et pouvait être recyclée pour une autre opération, moyennant un ajout d'eau si nécessaire.

L'acide chlorhydrique abattu titrait 33 % et ne contenait pas de traces détectables de composés organiques. Vu sa très faible teneur en d'acide sulfurique (37 ppm), il était propre à la commercialisation.

### Exemple 2 comparatif

On a opéré comme à l'exemple 1, mais sans ajouter d'eau et en introduisant directement le n-octylmercaptan dans le mélange réactionnel. Pour obtenir une conversion totale du mercaptan et des intermédiaires réactionnels en chlorure d'octanesulfonyle, le temps global de réaction est passé de six heures à neuf heures et a donc augmenté de 50 %.

La pureté du chlorure d'octanesulfonyle obtenu dans ces conditions n'était plus que de 92 % (production : 4013 g) et il contenait 4,65 % de chlorure de 1-chlorooctanesulfonyle, 1,3 % de chlorure d'octanesulfinyle et de son dérivé chloré, 1,2 % d'acide octanesulfinique et 0,5 % d'octanethiosulfonate d'octyle.

La pureté de l'acide chlorhydrique 33 % récupéré dans la colonne d'abattage est équivalente à celle de l'exemple 1.

### Exemple 3 comparatif

On a opéré comme à l'exemple 1, mais en remplaçant le pied de solution d'acide chlorhydrique 36 % par un pied d'eau pure de même masse (5200 g) et en introduisant le n-octylmercaptan seul (sans eau) dans le réacteur. Le temps de réaction total a été le même que celui de l'exemple 1.

La phase organique récupérée titrait 97,5 % en chlorure d'octanesulfonyle (soit 4230 g de chlorure d'octanesulfonyle) et ne contenait que 0,2 % de chlorure de 1-chloro-octanesulfonyle; les intermédiaires de synthèse (chlorure d'octanesulfinyle, acide octanesulfinique, octanethiosulfonate d'octyle) ne totalisaient que 1 %.

On a récupéré 7435 g de phase aqueuse acide titrant 40 % d'acide chlorhydrique et polluée par 2620 ppm d'acide sulfurique, 740 ppm d'acide octanesuifonique, 350 ppm de chlorure d'octanesulfonyle et une quantité non négligeable d'acide octanesulfinique qui n'a pas pu être dosée quantitativement.

La fraction pure d'acide chlorhydrique qui est parvenue à l'abattage à l'eau ne correspondait qu'à 18,7 % de l'acide total produit, soit 700 g donnant 2100 g de solution à 33 %, de pureté équivalente à celle de l'acide obtenu dans l'exemple 1.

La phase aqueuse chlorhydrique avait une composition voisine de la phase aqueuse résiduelle de l'exemple 1. On ne peut donc pas disposer des 81,3 % de l'acide chlorhydrique de la réaction sans procéder à une purification de ce dernier et donc augmenter son prix de revient.

Le tableau suivant dans lequel COS désigne le chlorure de n-octanesulfonyle et ClCOS le chlorure de 1-chloro-octanesulfonyle, résume les principaux résultats obtenus dans les exemples précédents pour une production de 4362 g de COS brut.

| Exemple | Pureté du COS (% massique) | Teneur en ClCOS (% massique) | Production d'acide chlorhydrique 33% pur |
|---|---|---|---|
| 1 | 97 | 0,25 | 9981 g |
| 2 | 92 | 4,65 | 11460 g |
| 3 | 97,5 | 0,20 | 2100 g |

**Exemples 4, 5 et 6 : Préparation des chlorures de butanesulfonyle (CBS), d'hexanesulfonyle (CHS) et de décanesulfonyle (CDS)**

On a opéré comme à l'exemple 1 avec prémélange du mercaptan et de l'eau avant de les injecter dans le milieu réactionnel, mais en remplaçant le n-octylmercaptan par la même quantité (3000 grammes) de n-butylmercaptan, de n-hexylmercaptan ou de n-décylmercaptan respectivement. Dans tous les cas, le pied d'eau était de 5200 grammes.

Les résultats obtenus sont rassemblés dans le tableau suivant qui indique la production de chlorure d'alcanesulfonyle brut, sa pureté et sa teneur en dérivé $\alpha$-chloré, ainsi que la production d'acide chlorhydrique 33 % de qualité commerciale.

| Exemple | Chlorure d'alcanesulfonyle | | | Teneur en $\alpha$-chloré (%) | Production d'acide chlorhydrique 33% pur |
|---|---|---|---|---|---|
| | nature | production | pureté (%) | | |
| 4 | CBS | 5207 g | 98 | 0,12 | 16900 g |
| 5 | CHS | 4685 g | 97,3 | 0,16 | 12622 g |
| 6 | CDS | 4143 g | 97 | 0,30 | 8189 g |

**Revendications**

1. Procédé de fabrication d'un chlorure d'alcanesulfonyle $RCH_2$-$SO_2Cl$ par chlorolyse oxydante du mercaptan $RCH_2$-SH correspondant, **caractérisé en ce que** le mercaptan et de l'eau sont introduits dans le mélange réactionnel sous forme d'une dispersion d'eau dans le mercaptan, le dit mélange réactionnel étant initialement composé d'une solution aqueuse concentrée d'acide chlorhydrique.

2. Procédé selon la revendication 1 dans lequel la quantité d'eau dispersée dans le mercaptan est telle que le rapport molaire eau : mercaptan soit compris entre 0,5:1 et 5:1 et, de préférence, égal à 2:1.

3. Procédé selon la revendication 1 ou 2 dans lequel le mélange réactionnel est initialement composé d'une solution ayant une teneur en HCl de 33 à 40 %.

4. Procédé selon l'une des revendications 1 à 3 dans lequel le chlore est introduit dans un rapport molaire chlore : mercaptan égal à 3:1.

5. Procédé selon l'une des revendications 1 à 4 dans lequel l'alkylmercaptan $RCH_2$-SH contient de 2 à 12 atomes de carbone, de préférence de 4 à 10.

6. Procédé selon la revendication 5 dans lequel le radical $RCH_2$ est un radical alkyle linéaire.

7. Procédé selon l'une des revendications 1 à 6 dans lequel le chlorure d'alcanesulfonyle produit est le chlorure de n-octanesulfonyle.

**Patentansprüche**

1. Verfahren zur Herstellung eines Alkansulfonylchlorids RCH$_2$-SO$_2$Cl durch oxidative Chlorolyse des entsprechenden Mercaptans RCH$_2$-SH, **dadurch gekennzeichnet, daß** man das Mercaptan und Wasser in Form einer Dispersion von Wasser in dem Mercaptan in die Reaktionsmischung einträgt, wobei die Reaktionsmischung anfänglich aus einer konzentrierten wäßrigen Salzsäurelösung besteht.

2. Verfahren nach Anspruch 1, bei dem die Menge des in dem Mercaptan dispergierten Wassers so gewählt wird, daß das Wasser/Mercaptan-Molverhältnis zwischen 0,5:1 und 5:1 liegt und vorzugsweise gleich 2:1 ist.

3. Verfahren nach Anspruch 1 oder 2, bei dem die Reaktionsmischung anfänglich aus einer Lösung mit einem HCl-Gehalt von 33 bis 40% besteht.

4. Verfahren nach einem der Ansprüche 1 bis 3, bei dem man das Chlor in einem Chlor/Mercaptan-Molverhältnis von 3:1 einträgt.

5. Verfahren nach einem der Ansprüche 1 bis 4, bei dem das Alkylmercaptan RCH$_2$-SH 2 bis 12 Kohlenstoffatome und vorzugsweise 4 bis 10 Kohlenstoffatome enthält.

6. Verfahren nach Anspruch 5, bei dem es sich bei dem RCH$_2$-Rest um einen linearen Alkylrest handelt.

7. Verfahren nach einem der Ansprüche 1 bis 6, bei dem man als Alkansulfonylchlorid-Produkt n-Octansulfonylchlorid erhält.

**Claims**

1. Process for the manufacture of an alkanesulphonyl chloride RCH$_2$-SO$_2$Cl by oxidative chlorolysis of the corresponding mercaptan RCH$_2$-SH, **characterized in that** the mercaptan and water are introduced into the reaction mixture in the form of a dispersion of water in the mercaptan, the-said reaction mixture being initially composed of a concentrated aqueous hydrochloric acid solution.

2. Process according to Claim 1, in which the amount of water dispersed in the mercaptan is such that the water: mercaptan molar ratio is between 0.5:1 and 5:1 and preferably equal to 2:1.

3. Process according to Claim 1 or 2, in which the reaction mixture is initially composed of a solution having an HCl content of 33 to 40%.

4. Process according to one of Claims 1 to 3, in which the chlorine is introduced in a chlorine:mercaptan molar ratio equal to 3:1.

5. Process according to one of Claims 1 to 4, in which the alkyl mercaptan RCH$_2$-SH comprises from 2 to 12 carbon atoms, preferably from 4 to 10.

6. Process according to Claim 5, in which the RCH$_2$ radical is a linear alkyl radical.

7. Process according to one of Claims 1 to 6, wherein the manufactured alkanesulphonyl chloride is n-octanesulphonyl chloride.